# EUROPEAN PATENT APPLICATION

(11) **EP 4 681 756 A1**
(43) Date of publication of application: **21.01.2026**
(21) Application number: 24188799.1
(22) Date of filing: 16.07.2024
(51) Int. Cl.: A61M 16/04

(54) **TRACHEOSTOMY TUBE WITH INTEGRATED CAMERA**

(71) Applicant: Ambu A/S, 2750 Ballerup (DK)
(72) Inventor: RASK, Jesper Domino, 2300 København S (DK); VILHELMSEN, Erik Øllgaard, 3460 Birkerød (DK)
(74) Representative: Winter, Brandl - Partnerschaft mbB

(57) **Abstract**

A tracheostomy tube includes a cannula including a wall forming a ventilation lumen, the cannula further including a curved section, a distal anterior side, a distal posterior side, and an open camera cord channel; a mounting plate; a cuff positioned distally of the curved section and extending over a portion of the camera cord channel; a cuff inflation lumen; a camera assembly positioned distally of the cuff; a front wall comprising an opaque portion and a transparent portion, the transparent portion positioned distally and in front of the camera assembly; a fluid lumen configured to supply a fluid to clean the transparent portion of the front wall; and a camera cord electrically connected to the camera assembly, the camera cord including a camera cord proximal portion positioned in the camera cord channel and a camera cord distal portion positioned distally of the cuff.

## Description

### CROSS-REFERENCE TO RELATED APPLICATIONS

None.

### TECHNICAL FIELD

The disclosure relates to a tracheostomy tube with a camera. The disclosure also relates to tracheostomy system including the tracheostomy tube and a video processor operable to receive images from the camera.

### BACKGROUND

Tracheostomy is a procedure in which a tracheostomy tube is inserted through an opening in the trachea to oxygenate a patient's lungs. After the tracheostomy the patient breathes through the tracheostomy tube. Tracheostomy tubes are typically used when a patient needs help breathing over long periods of time. A tracheostomy tube may include a cuff provided to immobilize the tracheostomy tube in the trachea of the patient.

An early example of a tracheostomy tube assembly is disclosed in U.S. Pat. No. 5,056,515. As described in the abstract, the tracheostomy tube assembly includes a short, curved outer cannula affixed to a neck plate with strap, and a removable inner cannula having an affixed inflatable endotracheal cuff. When in use, the endotracheal cuff of the inner cannula is positioned beyond the terminal end of the outer cannula within the trachea.

Another example of a tracheostomy tube is disclosed in U.S. Patent Publication No. 2019/0059710. As described in the abstract, the publication discloses medical devices comprising a camera combined with a second device selected from an endotracheal tube, oral airway, supraglottic airway, tracheostomy tube, suction catheter, tubeless intubating device, tool tube and/or stylet.

A laryngeal tube with an inflatable cuff is also described in commonly-owned U.S. Pat. No. 9,421,341. An endobronchial tube with an inflatable cuff and an image sensor is described in commonly-owned U.S. Pat. No. 10,245,402.

One problem with current tracheostomy tubes is that they may be uncomfortable. Because the tracheostomy tubes are used over long periods, pressure on tissue and friction caused by movement can cause discomfort when such would be unnoticed over short periods of time. Such problems may be even more noticeable when the patient is ambulatory. Another problem is that a tracheostomy tube may move to an undesired position or the cuff may disinflate, and thus the patient may receive less oxygen than when the tracheostomy tube is first positioned in the patient.

### SUMMARY

The present disclosure provides solutions which at least improve the tracheostomy tubes of the prior art. In some aspects of the disclosure, solutions are provided to present on a display images indicative of the position of the tracheostomy tube, the condition of the trachea, and/or the presence of obstructions or secretions. A physician can then confirm that the placement and ventilation are adequate. A physician can also monitor the tracheostomy site to detect early signs of complications or changes in the patient's condition. This could include identifying issues such as bleeding, granulation tissue formation, or infections before they become severe. Other aspects provide monitoring mechanisms. The monitoring mechanisms can alert when the cuff is disinflated. With continuous monitoring, healthcare providers may be able to reduce the frequency of invasive procedures, like bronchoscopy, which is typically used to assess the tracheostomy site. In telemedicine or remote healthcare scenarios, the camera can allow healthcare providers to assess and guide tracheostomy care from a distance. For patients, the presence of a camera may offer peace of mind as they can see the condition of their tracheostomy and can better understand the need for any interventions.

A tracheostomy tube is provided in first aspect of the invention.

In a first embodiment according to the first aspect, the tracheostomy tube includes a cannula including a wall forming a ventilation lumen, the cannula further including a curved section, a distal anterior side, a distal posterior side, and a camera cord channel; a mounting plate; a cuff positioned distally of the curved section and extending over a portion of the camera cord channel; a cuff inflation lumen; a camera assembly positioned distally of the cuff; a front wall comprising an opaque portion and a transparent portion, the transparent portion positioned distally and in front of the camera assembly; a fluid lumen configured to supply a fluid to clean the transparent portion of the front wall; and a camera cord electrically connected to the camera assembly, the camera cord including a camera cord proximal portion positioned in the camera cord channel and a camera cord distal portion positioned distally of the cuff. The camera cord channel may be open or it may be closed.

In a variation of the present embodiment, the camera assembly further comprises a camera housing, the camera housing comprising the front wall, and the cannula comprising a camera housing cavity, wherein the camera housing is positioned in the camera cavity and the camera assembly is positioned in the camera housing. The camera housing may comprise a discorectangular shape.

In another variation of the present embodiment, the tracheostomy tube further comprises a tip part, the tip part comprising a camera cavity, a ventilation lumen extending from the ventilation lumen of the cannula, and the front wall, wherein the camera assembly is positioned in the camera cavity of the tip part.

In another variation of the present embodiment, the camera cord proximal portion is radially offset from the camera cord distal portion by between 30 and 120 degrees.

In another variation of the present embodiment, the camera cord comprises a relief portion.

In another variation of the present embodiment, the tracheostomy tube comprises a cuff pressure sensor. The cuff pressure sensor may be positioned between the cannula and the cuff. Ends of the cuff may be secured to the cannula proximally and distally of the pressure sensor to enable the pressure sensor to sense pressure in the cuff.

A video processor is provided in a second aspect of the invention.

In one embodiment according to the second aspect, the video processor is configured to perform a pressure monitoring method comprising: periodically or continuously receiving a pressure signal from a pressure sensor comprised in a tracheostomy tube; comparing the pressure signal to the pressure range, and outputting a warning or alarm signal if the pressure signal is indicative of a cuff pressure being outside the pressure range.

A monitoring system is provided in a third aspect of the invention.

In an embodiment according to the third aspect, the monitoring system comprises a tracheostomy tube and a video processor. The tracheostomy tube may comprise the tracheostomy tube according to the first embodiment of the first aspect and any variations and examples thereof described below. The video processor may comprise the video processor according to the second aspect.

In one embodiment according to the third aspect, the video processor is configured to perform a pressure monitoring method comprising: periodically or continuously receiving a pressure signal from a pressure sensor comprised in the tracheostomy tube; comparing the pressure signal to the pressure range, and outputting a warning or alarm signal if the pressure signal is indicative of a cuff pressure being outside the pressure range.

In one embodiment according to the second and/or third aspects, the warning or alarm signal comprises a Bluetooth signal transmitted to a paired mobile device.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

The disclosure will be described in more detail below with reference to the following figures. The figures illustrate embodiments, variations and examples of the invention to facilitate the understanding of a person of ordinary skill in the art and are not to be construed as limiting the scope of the claims.
FIG. 1 is a schematic illustration of a tracheostomy system including a tracheostomy tube;
FIG. 2 is a schematic perspective view of an embodiment of a tracheostomy tube;
FIG. 3 is a sectional side view of the embodiment of the tracheostomy tube of FIG. 2;
FIG. 4 is a sectional side view of a variation of the embodiment of the tracheostomy tube of FIGS. 2 and 3;
FIGS. 5 and 6 are distal views of variations of the embodiment of the tracheostomy tube of FIGS. 2 and 3, showing camera assembly positions;
FIG. 7 is a side view of a variation of the embodiment of the tracheostomy tube of FIGS. 2 and 3 showing a distal tip;
FIGS. 8 to 10 are distal views of further variations of the embodiment of the tracheostomy tube of FIGS. 2 and 3, showing camera assembly positions;
FIGS. 11 to 15 are views including perspective, exploded, proximal and distal, of further variations of the embodiment of the tracheostomy tube of FIGS. 2 and 3, showing a discorectangular camera assembly and distal tip with a camera assembly without a separate housing;
FIGS. 16 and 17 are side perspective and assembled views of an example camera assembly;
FIGS. 18 and 19 are views of embodiments of channels in the cannula of a tracheostomy tube according with the present disclosure;
FIGS. 20 and 21 are views of an embodiment of a camera cord routing in the cannula of a tracheostomy tube according with the present disclosure;
FIG. 22 is a view of another embodiment of a camera cord routing in the cannula of a tracheostomy tube according with the present disclosure;
FIG. 23 is a front view of a video processor;
FIG. 24 is a block diagram of an image processing circuit of the video processor of FIG. 23; and

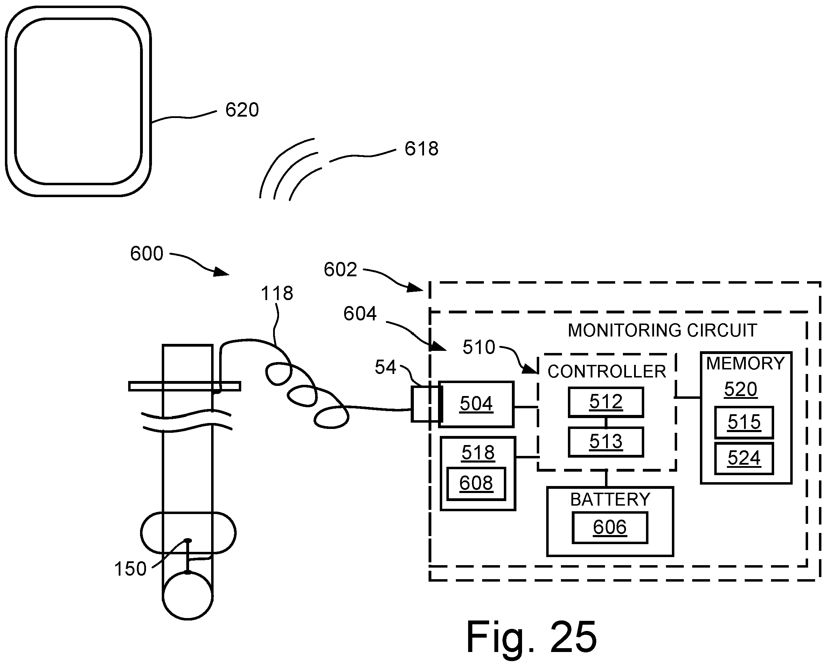

FIG. 25 is a block diagram of a monitoring system.

### DETAILED DESCRIPTION OF SELECTED EMBODIMENTS

In the following description, for purposes of explanation, specific details are set forth in order to provide an understanding of the invention. It will be apparent, however, to one skilled in the art that the invention can be practiced without all these details. Furthermore, one skilled in the art will recognize that embodiments described below may be implemented in a variety of ways, such as a process, an apparatus, a system, a device, or a method.

Connections between components or systems within the figures are not intended to be limited to direct connections. Rather, data between these components may be modified, re-formatted, or otherwise changed by intermediary components. Also, additional or fewer connections may be used. It shall also be noted that the terms "coupled," "connected," or "communicatively coupled" shall be understood to include direct connections, indirect connections through one or more intermediary devices, and wireless connections.

Reference in the specification to "one embodiment," "preferred embodiment," "an embodiment," or "embodiments" means that a particular feature, structure, characteristic, or function described in connection with the embodiment is included in at least one embodiment and may be in more than one embodiment. Also, the appearances of the above-noted phrases in various places in the specification are not necessarily all referring to the same embodiment or embodiments. Furthermore, the use of certain terms in various places in the specification is for illustration and should not be construed as limiting. Any headings used herein are for organizational purposes only and shall not be used to limit the scope of the description or the claims.

Furthermore, it shall be noted that: (1) certain steps may optionally be performed; (2) steps may not be limited to the specific order set forth herein; (3) certain steps may be performed in different orders; and (4) certain steps may be done concurrently.

The disclosures of the following documents are incorporated herein by reference in their entirety: U.S. Patent No. 11,291,352, U.S. Pat. No. 11,786,108, and European Patent Application No. 24 154 698.5, filed January 30, 2024.

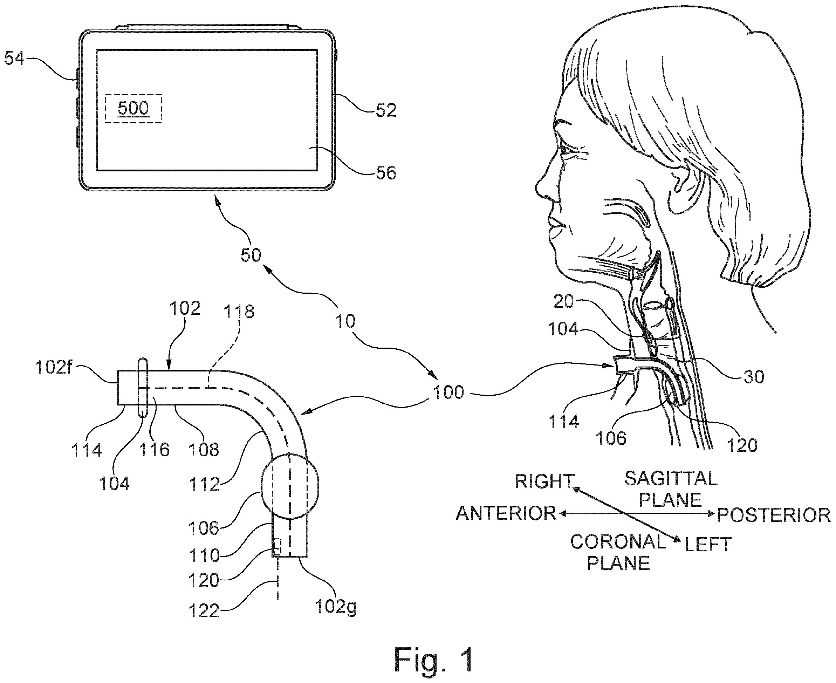

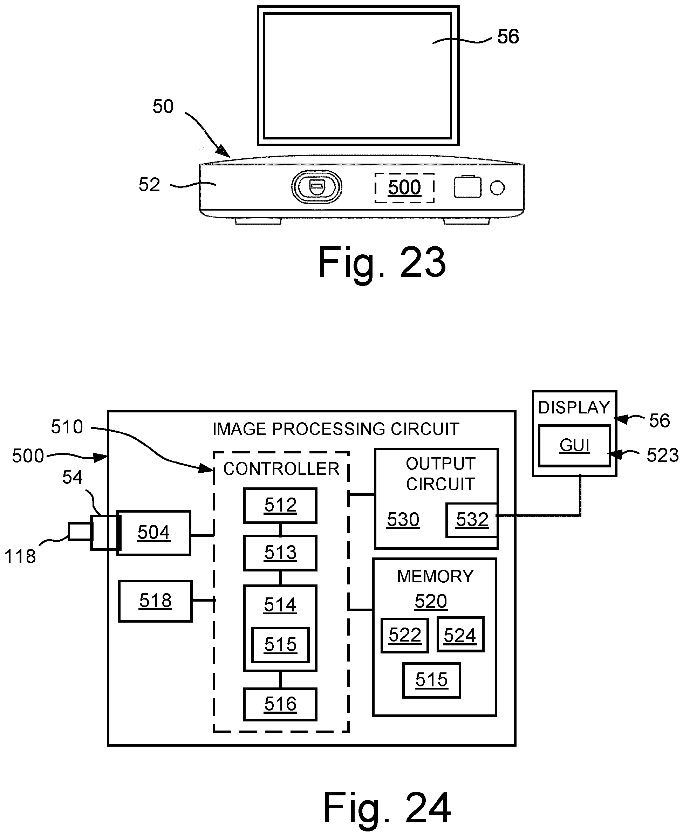

FIG. 1 is a schematic illustration of a tracheostomy system 10 including a tracheostomy tube 100 positioned in the trachea 20 of a patient/wearer and a video processor 50, described in more detail with reference to FIGS. 23 and 24. The esophagus 30 of the wearer is also shown. The tracheostomy tube 100 is inserted through a tracheostomy opening in the trachea 20.

Standard anatomical terms are used herein when referring to the body of the wearer. The sagittal and coronal planes are orthogonal and vertical relative to a standing wearer. The sagittal plane extends in the anterior-posterior direction (belly-back) and the coronal plane traverses the shoulders. Thus, the tracheostomy tube 100 is inserted through the tracheostomy opening along the sagittal plane. The terms "right" and "left" refer to the sides where the right and left hands of the wearer are located on the coronal plane relative to the intersection of the sagittal and coronal planes. Thus, when a figure shows the face of a standing wearer, the right hand of the wearer is on the left side of the figure.

The standard anatomical terms are used with reference to an inserted (in-use) tracheostomy tube. However, when referring to the tracheostomy tube, the term "distal" refers to the end of the tracheostomy tube that is inserted into the wearer and the term "proximal" refers to opposite end, which protrudes externally from the tracheostomy opening.

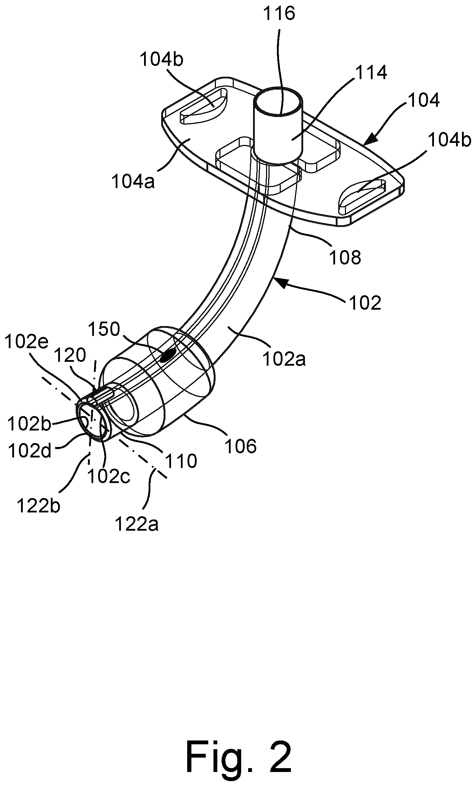

In the present embodiment, the tracheostomy tube 100 comprises a cannula 102 including a proximal end 108, a distal end 110, an outer surface 102a (FIG. 2), an inner surface 102b (FIG. 2), a distal anterior side 102c (FIG. 2), a distal posterior side 102d (FIG. 2), and a wall 102e extending from the proximal end 108 to the distal end 110 and forming a ventilation lumen 116; a mounting plate 104 positioned at the proximal end of the cannula 102; a cuff 106 positioned at the distal end of the cannula 102; a camera assembly 120 positioned distally of the cuff 106; and a camera cord 118 electrically connected to the camera assembly 120. A curved section 112 extends between the proximal end 108 and the distal end 110.

The camera assembly 120 comprises an optical axis 122 extending through a field of view directed distally of the cuff 106.

The cuff 106 is inflatable and is positioned distally of the curved section 112.

The mounting plate 104 abuts the throat of the wearer and may be secured thereto with a strap having its ends connected to two openings 104b (FIG. 2) in the mounting plate 104. A connector 114 extends proximally from mounting plate 104. The connector 114 can be integrated with the mounting plate 104 or be part of the proximal end 108 or be a separate part. A ventilator circuit (not shown) may be connected to the connector 114 at the proximal end 108 to ventilate the patient.

The video processor 50 comprises a housing 52, a communication port 54, illustratively shown as a hardwired receptacle, and an image processing circuit 500 (FIG. 24). The communication port 54 may, optionally or additionally, comprise a wireless transceiver. The video processor 50 is configured to present images captured by the camera assembly 120 on a display 56, illustratively shown as an integrated display within the housing 52. The display 56 may, optionally or additionally, be communicatively connected but be separate or separable from, in normal use, the housing 52, as shown in FIG. 23. For example, the display 56 may comprise an existing wall-mounted display while the video processor 50 may be a portable medical device communicatively connected to the display 56 with a video cord extending from the housing 52 or wirelessly.

FIG. 2 is a schematic perspective view of the embodiment of the tracheostomy tube 100 of FIG. 1. As shown, the mounting plate 104 includes a plate 104a comprising the strap openings 104b on the left and right sides, respectively, of the plate 104a.

A pressure sensor 150 may be provided to sense the cuff pressure. Particularly when the tracheostomy tube 100 is positioned for extended use, during which time the cuff 106 may lose cuff pressure, the cuff pressure sensed by the pressure sensor 150 can be used by the video processor 200 to monitor the cuff pressure and output a cuff pressure alarm when the cuff pressure reaches or falls below a predetermined pressure threshold. The cuff pressure can also be used to inflate the cuff 106 to a desired pressure. A pressure of 20-30 cm H₂O is generally considered optimum for the cuff. A lower pressure may increase the risk of leaks, so the patient does not receive the proper ventilation, and may also increase the risk of ventilator acquired pneumonia due to aspiration. A higher pressure, such as higher than 35 cm H₂O, may increase the risk of tracheal injury, dilation of the trachea, tissue necrosis or cuff rupture. Manual pressure check involving connecting the cuff to a cuff pressure manometer may, however, involve a risk of pressure loss due to the measurement, so the actual cuff pressure in the cuff is lower than measured. The pressure sensor 150 mitigates and/or overcomes these problems.

Also shown is a distal anterior/posterior plane 122a and a distal transverse plane 122b orthogonal to the distal anterior/posterior plane 122a. As shown, the camera assembly 120 is positioned on the distal anterior side 102c, and more particularly, on the distal transverse plane 122b. The camera assembly 120 may also be positioned on the distal anterior side 102c but offset from the distal transverse plane 122b. Whereas the position shown may be referred to as 12 o'clock, the distal anterior side 102c may be said to extend from 9 to 3 o'clock including 12 o'clock, and the distal posterior side 102d may be said to extend from 9 to 3 o'clock excluding 12 o'clock.

### 1. CAMERA PLACEMENT / POSITIONING

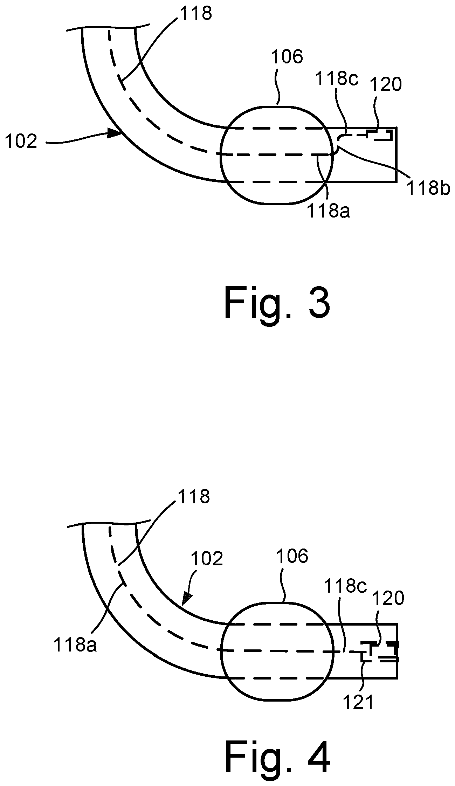

FIG. 3 is a sectioned side view of the embodiment of the tracheostomy tube 100 of FIG. 2 and FIG. 4 is a sectioned side view of a variation of the embodiment of the tracheostomy tube of FIGS. 2 and 3. A difference between the variations of the tracheostomy tube 100 shown in FIGS. 3 and 4 is the position of the camera assembly 120. In FIG. 3, the camera assembly 120 is positioned on the distal anterior side 102c, and more particularly on the distal transverse plane 122b, whereas in FIG. 4 the camera assembly 120 is positioned on the the distal anterior/posterior plane 122a. Positioning the camera assembly 120 on the distal anterior/posterior plane 122a has the benefit that there is a low risk of the camera contacting the trachea wall during insertion. Positioning the camera assembly 120 on the right side has the benefit that the camera may provide a better view into the lung due to the asymmetry of the trachea. In another variation mentioned above, the camera assembly 120 is positioned between 9 and 12 o'clock (on the right side of the distal transverse plane 122b), thereby reducing the risk of the camera contacting the trachea wall during insertion while providing a better view than if the camera were on the distal anterior side 102c and the distal transverse plane 122b.

FIGS. 3 and 4 also show the camera cord 118. The camera cord 118 is preferably and primarily routed on a lateral (right or left) side of the cannula 102. Thus, in FIG. 3 the camera cord 118 comprises a camera cord proximal portion 118a extending proximally from the cuff 106, a camera cord distal portion 118c extending distally of the cuff 106, and a camera cord traversing portion 118b extending between the camera cord proximal portion 118a and the camera cord distal portion 118c.

In FIG. 4, the camera assembly 120 is positioned on the distal anterior/posterior plane 122a (on the right side) and the camera cord 118 is devoid of the camera cord traversing portion 118b. The camera cord proximal portion 118a and the camera cord distal portion 118c extend along a lateral side, which is simpler to manufacture. The camera assembly 120 is positioned in a camera housing cavity 121.

In another variation, the camera assembly 120 is positioned between 9 and 12 o'clock, thereby reducing the risk of the camera contacting the trachea wall during insertion while providing a better view than if the camera were on the concave side. Thus, the camera cord proximal portion 118a extends to the camera cord traversing portion 118b, which extends to the camera cord distal portion 118c, but the camera cord traversing portion 118b does not traverse as much as in FIG. 3 (less than 90 degrees).

### 2. CAMERA MOUNTING

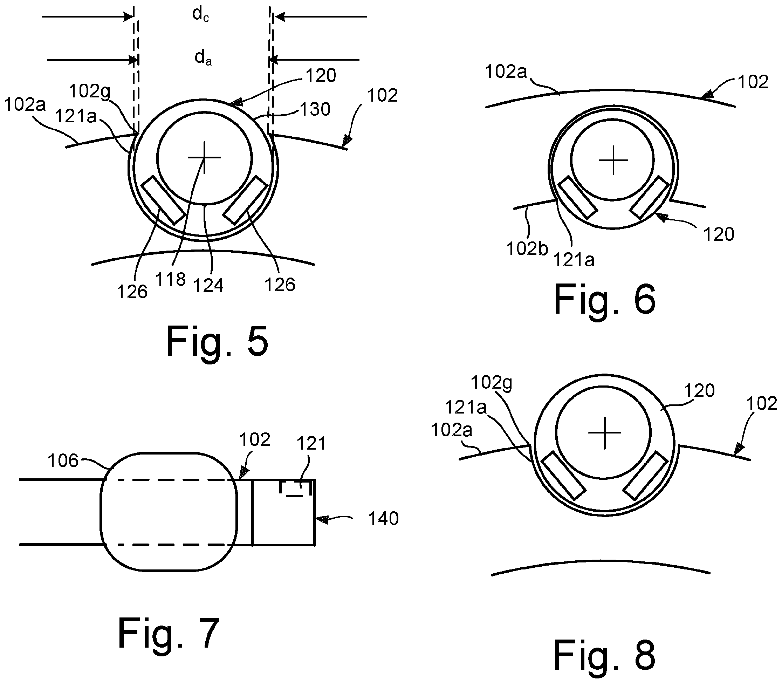

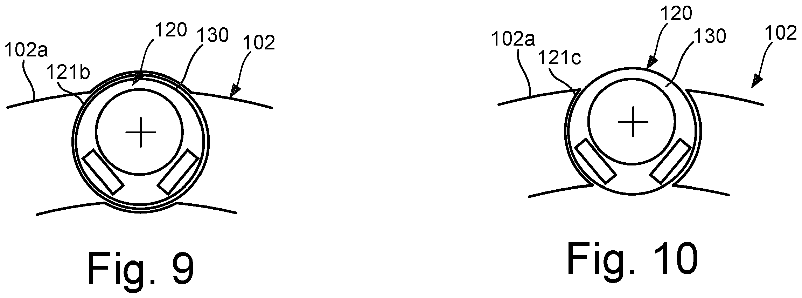

The camera assembly 120 may be positioned in the tracheostomy tube 100 in several ways. FIGS. 5 to 9 illustrate various ways to position the camera assembly 120 in the camera housing cavity 121 of the tracheostomy tube 100. The camera housing cavity 121 may be referred to as a semi-open cavity 121a (FIGS. 5, 6 and 8), a closed cavity 121b (FIG. 9), and a slot 121c (FIG. 10). As shown in FIGS. 5, 6, and 8-12, the camera assembly 120 comprises a camera 124 having the optical axis 118, a pair of light emitting diodes (LEDs) 126, and a housing 130 having a cross-section width d_{c}. When the housing 130 has a cylindrical shape, the cross-section width d_{c} is the diameter of the cylinder. However, the housing can comprise any cross-section shape, including rectangular, oval and discorectangle, among others. The discorectangle shape, also known as pill, stadium, or sausage shape, comprises a rectangle with semicircles at a pair of opposite sides. A width extends between the semicircles and a thickness extends between to sides of the rectangle. The discorectangle shape is advantageous because it allows for distribution of components along the camera assembly's width to minimize its thickness. The sides of the rectangle between the semicircles can be straight, as the term rectangle implies, but the sides can also be curved. Advantageously, the sides can be curved to approximate or match the curvature of the cannula 102.

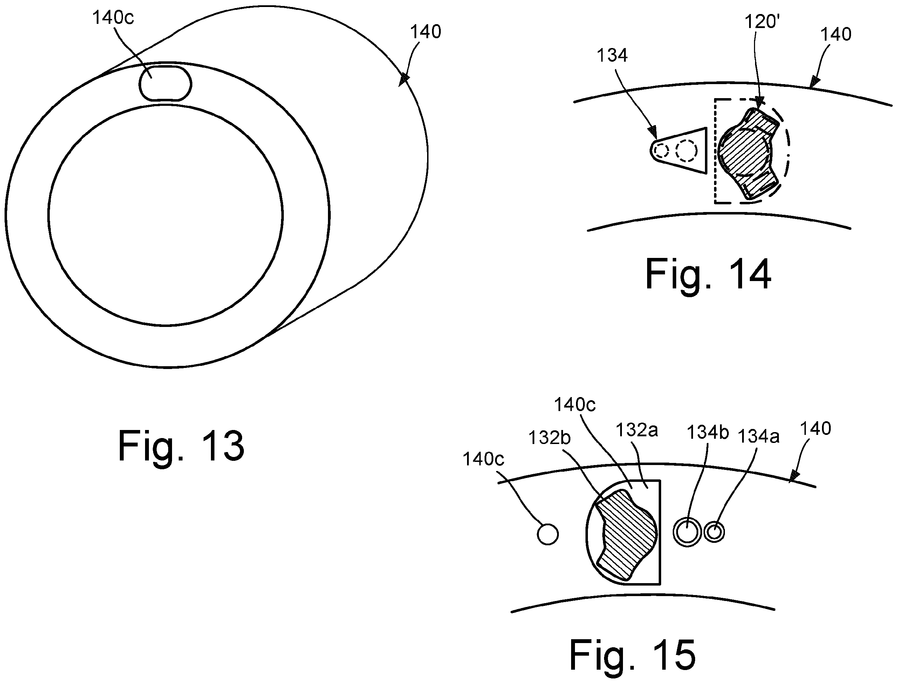

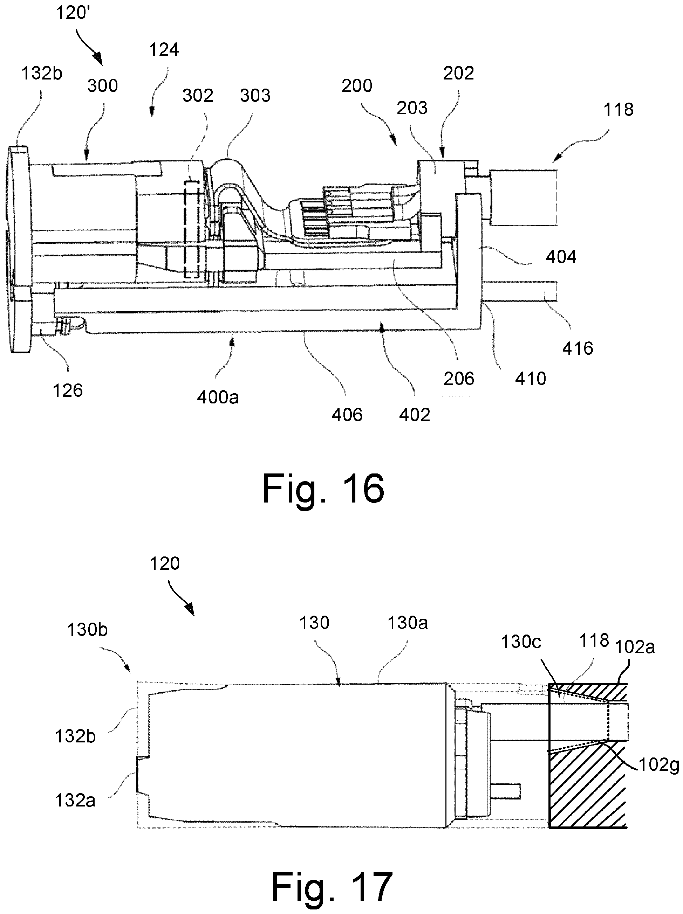

The camera housing cavity 121 (i.e. 121a, 121b, 121c) may be located in the distal end 110 of the cannula 102, as shown in FIGS. 5, 6, and 8-10. Alternatively or additionally, a distal tip 140 (FIG. 7) may be attached to the distal end 110 of the cannula 102 and the distal tip 140 may comprise, at least in part, the camera housing cavity 121. Preferably, the distal tip 140 is comprised of polymers. The distal tip may be molded and be rigid, e.g. inflexible. An advantage of using a distal tip is that the cannula 102 can be extruded, which is an economical process, and the distal tip, which is more complex in shape, can be injection molded, which is a process that lends itself to produce three-dimensional structures economically. The cavities shown in the distal end 110 of the cannula 102, as shown in FIGS. 5, 6, and 8-10, may, alternatively, be positioned in the distal tip 140. The distal end of the cannula and the proximal end of the distal tip may be chamfered to facilitate assembly, so one fits into the other, and ultrasonic and/or adhesive bonding to secure the distal tip to the cannula. The camera assembly may have a proximal "tip" or protrusion that extends proximally and penetrates the wall of the cannula so as to align, radially, the camera with the cannula and also to ensure a more secure assembly. The proximal tip may be hollow and the camera cord may protrude proximally through the hollow proximal tip, whereby the hollow proximal tip surrounds the camera cord for one or more millimeters. The wall may comprise a hole, extending proximally from an edge of the wall, sized to receive the proximal tip of the camera assembly. An example of a proximal tip 140c is shown in FIG. 15 extending proximally from the distal tip 140. The proximal tip 140c is not hollow but it could be. An example of a hollow proximal tip 130c is shown in FIG. 17 extending proximally from the housing 130. The wall 102a of the canula 102 is shown, in cross-section, receiving the hollow proximal tip 130c in a cavity 102g. The proximal tip can also be the fluid channel 134a and/or the fluid channel 134b, which may extend from the front wall and be connected to respective tubing within the housing 130 or may extend proximally and receive the tubing.

Referring to FIGS. 5 and 6, the wall 102e of the cannula 102 comprises a semi-open cavity 121a with an elongate gap 102g having a gap width dₐ smaller than d_{c}. The wall 102e is formed from polymers, preferably extruded and preferably resilient or flexible. Accordingly, when the camera assembly 120 is inserted in the semi-open cavity 121a through the gap 102g, the elongate gap 102g widens to receive the camera assembly 120 and thereafter returns to its original shape, thereby helping to secure the camera assembly 120 in the distal end 110 of the cannula 102. The camera assembly 120 may be adhesively bonded in the semi-open cavity 121a to further secure the camera assembly 120 to the cannula 102. Of course, the camera assembly 120 can also be inserted through a distal opening of the semi-open cavity 121a.

In FIG. 5 the elongate gap 102g is on the outer surface 102a of the cannula 102. In FIG. 6 the elongate gap 102g is on the inner surface 102b of the cannula 102.

In a variation of the present embodiment, shown in FIG. 8, the wall 102e of the cannula 102 comprises the semi-open cavity 121a. However, the elongate gap 102g does not have a gap width dₐ smaller than d_{c}. The gap width dₐ is equal to or greater than the width of the camera assembly 120. This structure facilitates insertion of the camera assembly 120, which may be adhesively bonded in the semi-open cavity 121a.

In a further variation of the present embodiment, the semi-open cavity 121a of FIG. 8 may be provided with the elongate gap on the inner surface 102b of the cannula 102.

In another variation of the present embodiment, shown in FIG. 9, the wall 102e of the cannula 102 comprises the closed cavity 121b configured to receive the housing 130. The camera assembly 120 may be inserted into the closed cavity 121b (or any camera housing cavity 121) from a distal end thereof. By "closed" it is meant that the cavity does not have an open slot that extends along the length (longitudinally) of the camera assembly 120. The closed cavity 121b comprises a distal opening, through which the housing 130 may be inserted, and may comprise a proximal opening.

In a further variation of the present embodiment, shown in FIG. 10, the wall 102e of the cannula 102 comprises a slot cavity 121c configured to receive the housing 130. The slot cavity 121c functions similarly as the semi-open cavity 121a, except that by having opposing gaps the camera assembly 120 can have a thickness greater than a thickness of the wall 102e without increasing the radial profile of the distal end as much as if the same camera assembly 120 were inserted in the cavities of FIGS. 5 and 6.

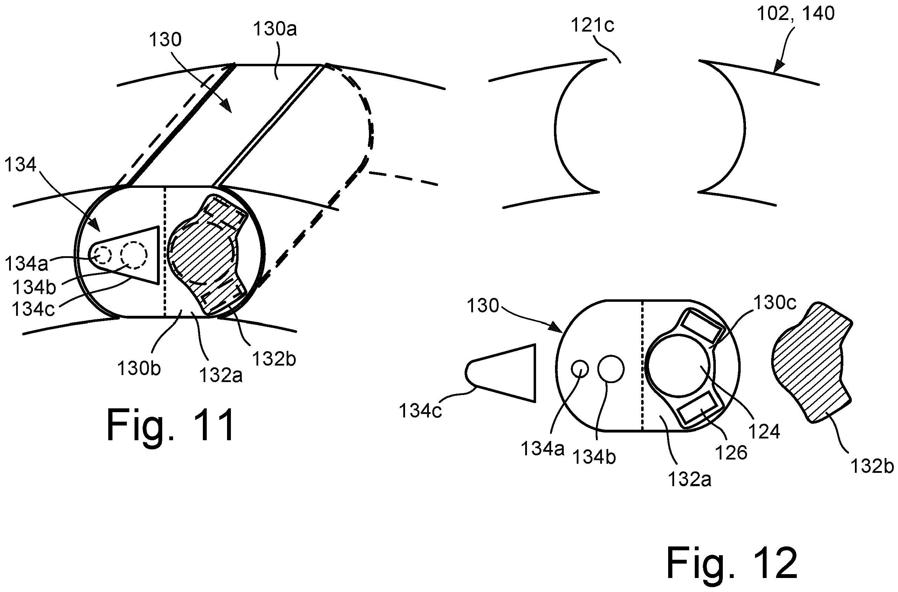

The camera assembly 120 may comprise a discorectangular radial (vs. longitudinal) cross-section, as shown in FIGS 11 and 12. FIG. 12 shows an exploded view of FIG. 11. In FIGS. 11 and 12 the camera assembly 120 is positioned in the slot cavity 121c first shown in FIG. 10. However, the same camera can be positioned in any of the camera cavities 121 described above, suitably modified to receive the discorectangular camera assembly 120. The discorectangular camera assembly 120 comprises the housing 130, the LEDs 126, and the camera 124. These components are positioned on one side of the housing 130. On the opposite side there is a nozzle 134. The camera assembly may be referred to, without the housing 130, as a camera assembly 120', which is discussed with reference to FIGS. 14 and 15.

The housing 130 comprises a longitudinal wall 130a, a front wall 130b at a distal end of the longitudinal wall 130a, and a camera cavity 130c formed by the longitudinal wall 130a and the front wall 130b. The front wall 130b comprises an opaque portion 132a and a transparent portion 132b. The housing 130 may be manufactured in a two-stage injection molding process described in U.S. Pat. No. 11,291,352. The components of the camera assembly may be as described in U.S. Pat. No. 11,291,352, may comprise a universal camera assembly as described in European Patent Application No. 24 154 698.5, filed January 30, 2024, and may comprise any other known camera assembly or module that can fit in the camera housing cavity 121.

The nozzle 134, as shown, comprises a fluid channel 134a, an optional fluid channel 134b, and a diverter cover 134c. The diverter cover comprises a diverter wall positioned distally of the distal ends of the fluid channels 134a, 134b and, optionally, spacer walls on both sides of the fluid channels 134a, 134b that define a gap between the diverter wall and the front wall 130b. Of course, the front wall can be curved with its edges contacting the front wall, the curvature defining the gap. A fluid passing through the fluid channels 134a, 134b splatters on an inner surface of the diverter wall and the splattered fluid, guided by the spacer walls toward the camera 124, flushes the surface of the front wall portion 132b covering the camera 124. The fluid may comprise a gas (e.g. air) and a liquid and be directed continuously or in a pulsing manner through one of the fluid channels 134a, 134b to optimize splatter and increase turbulent flow. Alternatively or additionally, a gas may pass through one fluid channel and a liquid through the other, the two mixing in the nozzle before being expelled toward the surface of the front wall portion 132b covering the camera 124 to flush and clean it. Additional fluid channels may be provided to optimize flushing and cleaning. The nozzle 134 may be injection molded with the housing or bonded onto the housing. The nozzle 134 may be manufacture and configured as described in U.S. Pat. No. 11,786,108.

As shown on FIG. 12, where the diverter cover 134c and the transparent front wall portion 132b are shown aside, the fluid channels 134a, 134b can be lumens molded in the housing and extending longitudinally in the housing 130. The housing 130 also includes the camera cavity 130c, where the camera assembly 120' is positioned. However, as shown in FIGS. 13-15, the camera assembly 120' can be positioned in a camera cavity 140c formed in the distal tip 140, whereby the camera housing 130 is omitted. Additionally, the nozzle 134 may be provided in the distal tip 140, as shown in FIG. 14. FIG. 15 is a proximal view (looking distally) of FIG. 14 with the camera assembly 120' removed, showing the fluid channels 134a, 134b, the opaque front wall portion 132a and the transparent front wall portion 132b. The nozzle 134 is not drawn to scale. Because the wall 102e has a thickness corresponding to the thickness of the distal tip 140, preferably the fluid channels 134a, 134b are as large as possible within the constraints of the thickness of the wall 102e and the distal tip 140 so as to minimize back-pressure. As shown in FIG. 15, the fluid channels 134a, 134b may form protruding tubes that can fit inside the fluid lumens in the wall 102e, e.g. lumens 142c, 142d. To insert the protruding tubes, the distal ends of the lumens 142c, 142d can be stretched by warming the cannula 102 and inserting a tool with conical points. Thereafter the protruding tubes can be pushed into the expanded distal ends of the lumens 142c, 142d.

A camera assembly 120' may be defined as a camera assembly 120 without a housing. Any known camera assembly 120' may be used. One suitable camera assembly 120 is shown in FIGS. 16 and 17 and described in European Patent Application No. 24 154 698.5. The universal camera assembly, as shown, comprises the LEDs 126, the camera 124, a sensor holder 200, and a lighting module 400a. The sensor holder 200 is not necessarily affixed to the first lighting module 400a and, therefore, can move, relative to the first lighting module 400a, in the anterior/posterior direction, longitudinally, and/or rotatably, so that alignment of the sensor holder 200 is independent of the alignment of the first lighting module 400a. During assembly, distal movement of the lighting module 400a stops when the LED 126 abuts the front wall portion 132b.

The camera 124 includes an image sensor 302 positioned in a barrel 300 comprising lenses, as is known in the art, and connected to circuit boards 303. The barrel 300 is attached to the sensor holder 200 and can move with it, potentially independently from the lighting module 400a. The sensor holder 200 comprises a cable holder 202 including an outer surface 203. The cable holder 202 is connected to a longitudinal bed 206 including a bed surface to which circuit boards 303 may be attached. The camera cord 118 passes through a recess in the cable holder 202.

The lighting module 400a comprises a body 402 including a proximal transverse wall 404 and a longitudinal bed 406 extending distally from the proximal transverse wall 404. The proximal transverse wall 404 comprises a cable recess 410 configured to receive a cable assembly 416 providing power to the LEDs 206. Alternatively, the cable assembly 416 may be electrically connected to the circuit boards 303 to receive power for the LEDs therefrom. The sensor holder 200 is mounted onto the lighting module 400a. Different lighting modules can be used with the camera 124 and sensor holder 200 to accommodate, for example, different lighting arrangements with and without working channels. The lighting arrangements could comprise a distal end of a fiberoptic cable instead of LEDs.

As described below, the above-described camera assembly 120' can be used with a tip part that functions as a housing and can also be used with a housing as shown above in FIGS. 5, 6, and 8-10.

### 3. CAMERA CORD ROUTING

The cannula 102 may comprise channels including a cord channel 142a, a cuff inflation channel 142b, a first fluid channel 142c, and a second fluid channel 142d. The channels may be open or closed. A closed channel may be referred to as a lumen. It should be understood that the lumen may be open at both ends but is closed along substantially its entire length.

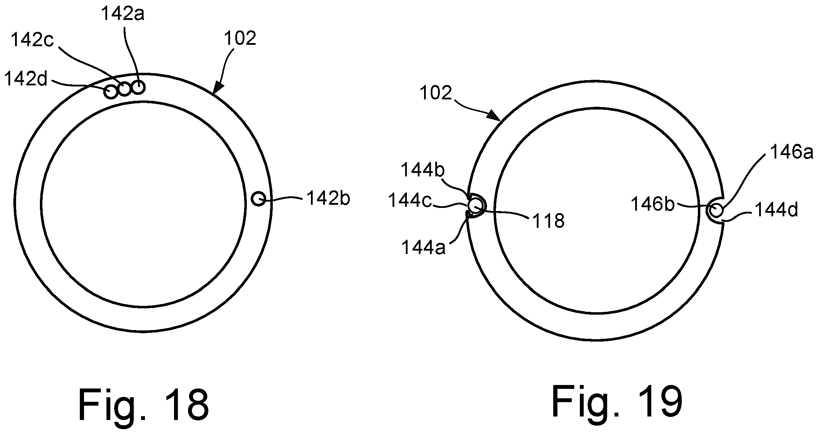

Examples of lumens are shown in FIG. 18, which is a radial cross-section of the cannula 102, which may be proximal of the cuff 106. The lumens include the cord lumen 142a, the cuff inflation lumen 142b, the first fluid lumen 142c, and the second fluid lumen 142d. The cord lumen 142a is configured to route the camera cord 118. The cuff inflation lumen 142b is configured to receive air and discharge the air under the cuff 106 to inflate it. To that end a hole may extend from the outer surface of the cannula 102 to the inflation lumen 142b under the cuff 106. The first fluid lumen 142c and the second fluid lumen 142d may be fluidly coupled with the fluid channels 134a, 134b to deliver fluids thereto. By fluidly coupled it is meant that the lumens and the channels may be connected, directly or indirectly, to pass fluids therebetween. Tubes can be attached to the proximal ends of the lumens to connect the lumens to fluid sources, for example, such as a hand pump or an electrical pump with a fluid reservoir.

The camera cord 118 may be threaded through the lumen or the lumen may be formed around the camera cord 118 when the cannula 102 is extruded.

An open channel, by contrast, comprises a longitudinal gap, or slit. A tube or the camera cord 11 may be inserted into the open channel through the longitudinal gap. The open channel may be formed when the cannula is extruded. Examples of open channels are shown in FIG. 19, with is a radial cross-section of the cannula 102, which may be proximal of the cuff 106. The open channel includes a restricted open channel 144a and an unrestricted open channel 144d. The restricted open channel 144a comprises a pair of lips 144b that narrow the width of a slit 144c so that it is narrower than a transverse length of the restricted open channel. Thereby, a tube or cord can be pressed into the restricted open channel 144a between the lips 144b and the lips 144b can then retain the tube or cord. The unrestricted open channel 144d is devoid of the narrow slit, therefore a tube or cord can fall out unless it is affixed thereto. A tube 146a is shown comprising a lumen 146b. Multiple of the open channels and tubes can be provided to hold the camera cord 118 and form the cuff inflation lumen 142b, the first fluid lumen 142c, and the second fluid lumen 142d. More than one tube can be provided in an open channel. If tubes are provided inside open channels to supply lumens, a single channel may contain more than one tube, or cord, to simplify manufacturing. For example, three tubes can be extruded together and placed in a single open channel, to simplify manufacturing, providing three lumens. At present it is considered advantageous to route the camera cord in an open channel provided on the outer surface 102a of the cannula 102, as this is particularly well suited for easy assembly and hence low cost of manufacturing.

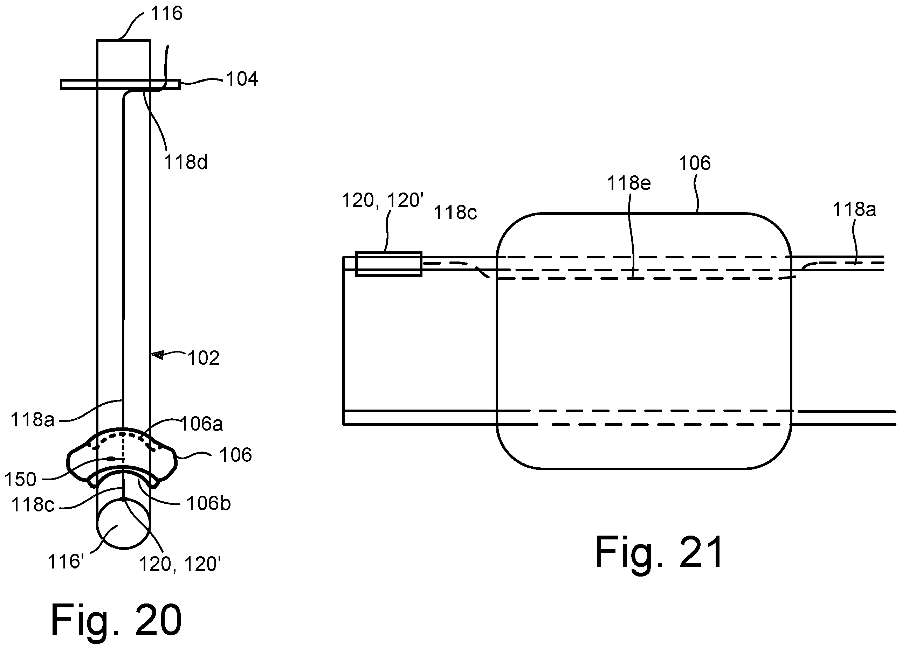

Referring now to FIG. 20, in one variation of the present embodiment the camera cord 118 extends along a concave side of the cannula 102. As shown, the camera assembly 120, 120' is positioned at the 12 o'clock position and the camera cord 118 extends proximally therefrom without a radial offset. Cuff bonding areas 106a and 106b are shown. Between the cuff bonding areas 106a and 106b there is an optional pressure sensor 150. Wires may extend from the camera cord 118 to the pressure sensor and these wires will then extend proximally to the proximal end of the cannula 102. The channels mentioned above are present but not shown. The camera cord 118 may be provided in a channel. Optionally, a relief portion 118d of the camera cord 118 extends radially (relative to the position of the camera assembly) to create an offset and/or a chicane that provides slack for the camera cord 118 so that it may translate in the channel, taking up the slack, without adding tension to the tracheostomy tube 100. The relief portion 118d may extend transversely (90 degrees to the length of the cannula) or at an angle between 0 and 90 degrees. As shown, the relief portion 118d is positioned at the proximal end 108 of the cannula 102 adjacent the mounting plate 104. The relief portion 118d may also be positioned at the distal end 108 of the cannula 102 adjacent the camera assembly 120'. The camera cord 118 may, optionally, be fixed or retained at the relief portion 118d or the mounting plate 104 e.g. by glue. An advantage of providing a relief portion 118d is that any pull in the cord will not transfer to the connection with the camera assembly 120', thereby protecting the connection from damage and ensuring proper functioning of the camera assembly 120'.

FIG. 21 is a sectional side view of an example of the tracheostomy tube 100 of FIG. 18, showing that an unchanneled portion 118e of the camera cord 118 is disposed between the camera cord proximal portion 118a and the camera cord distal portion 118c, which are provided in channels. The camera cord passes through the wall 102e of the cannula 102 into and out of the ventilation lumen 116 on opposite sides of the cuff 106. The portion in the ventilation lumen 116 is the unchanneled portion 118e. This arrangement ensures that there are no air leaks in the cuff associated with the camera cord 118.

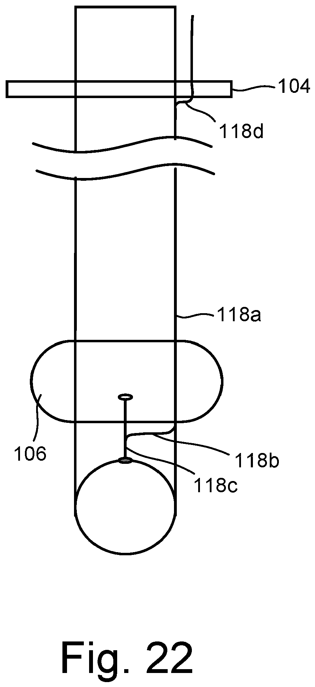

As described with reference to FIGS. 3 and 4, the camera cord 118 is preferably and primarily routed on a lateral (right or left) side of the cannula 102. Thus, in FIG. 22 the camera cord proximal portion 118a extends on a side, radially offset from the camera assembly 120', proximally from the cuff 106, a camera cord distal portion 118c extends distally of the cuff 106, potentially not radially offset from the camera assembly 120', and the camera cord traversing portion 118b extends between the camera cord proximal portion 118a and the camera cord distal portion 118c creating a radial offset between the camera cord proximal portion 118a and the camera cord distal portion 118c. The camera cord proximal portion 118a may be radially offset from the camera cord distal portion 118c by between 30 and 120 degrees. This amount of offset is sufficient to achieve the benefits of the desired camera location and manufacturing benefits due to the location of the camera cord proximal portion 118a.

FIG. 23 shows another embodiment of a video processor 50. The video processor 50 shown in FIGS. 1 and 23 comprise an image processing circuit 500 and may comprise a built-in display (FIG. 1) or a separate display (FIG. 23) that is not within the housing 52. Preferably, the video processor 50 is portable, meaning that it can be picked-up and held by a user. In other embodiments, the image processing circuit 500 can be integrated in a housing of another apparatus, such as a computer or a computer network. Variations of the video processor 50 can be provided with various features of the video processor 50 but including or excluding other features. For example, it might not be desirable to provide a display with a touch screen, or it might be desirable to omit a display altogether. Omission of the display might be beneficial to take advantage of evolving display technologies which improve resolution and reduce cost. Provision of exchangeable videoscope interfaces allows for adoption of evolving image sensor and videoscope technologies, thus use of existing or future-developed external displays could allow presentation of higher resolution or otherwise improved video. Use of external displays could also leverage existing capital investments. The video processor 50 is configured to present a live view corresponding to the images captured by the image sensor 302 shown in FIG. 16 or the image sensor of any camera assembly 120, 120'. As used herein, a videoscope comprises a device insertable into a patient and including a camera. Examples include the tracheostomy tube 100, endoscopes, laryngoscopes, endotracheal tubes, and the like.

The image processing circuit 500 is operable to receive image data, present a graphical user interface to allow a user to manipulate image data with the touch screen, and, optionally, output a video signal to allow remote viewing of the images presented with the display screen. A separate, potentially remote, display screen may also be connected to the endoscope via the video processor, which may include or omit the display screen. Medical device interfaces include circuits to compatibilize the signals from the image sensors, for example. Thus, a particular type of videoscope is matched with a corresponding medical device interface and the video processor can thus enable use of different videoscopes or other medical device technologies. In other words, the video processor is customized to work with the particular videoscope's technology.

FIG. 24 is a block diagram of an embodiment of the image processing circuit 500. The embodiment of the image processing circuit 500 depicted in FIG. 24 comprises a cable socket 54, a videoscope interface 504, a controller 510, a memory 520, and a video output board 530. One or more rigid circuit board parts may be provided to mount some or all the electronic parts, including the controller 510, the memory 520, and the video output board 530. The image processing circuit 500 interconnects the videoscope interface 504 with the controller 510, the memory 520, a user interface 518, and the video output board 530 in any manner known in the art. The image processing circuit 500 may comprise batteries and/or a power interface to receive power from a power source, such as a building's electrical system. The image processing circuit 500 may provide power to the image processing circuit 500 via the camera cord 118.

The videoscope interface 504 may include circuits to compatibilize, e.g. pre-process, the signals from the image sensor of the tracheostomy tube 100 to what the controller 510 expects to receive, in terms of image format, for example. Thus, a particular type of videoscope is matched with a corresponding videoscope interface and the video processor 50 can thus enable use of different videoscope technologies. The videoscope interfaces may also include isolation amplifiers to electrically isolate the video signal from the videoscope, and a power output connector to provide power to the videoscope for the image sensor and the LEDs. The videoscope interfaces may also include a serial to parallel converter circuit to deserialize the video signals of endoscopes that generate serial signals, for example serial video signals. The videoscope interfaces may also include analog to digital converters to digitize analog signals generated by the image sensor. In other words, the videoscope interfaces may be configured to receive analog or digital image signals. The videoscope interfaces may also comprise wireless transceivers to receive the image signals from the videoscope wirelessly. The videoscope interfaces may be removable so that various videoscopes may be used by inserting corresponding videoscope interfaces in the video processor. Multiple videoscope interfaces 504 may be provided to enable connections to multiple videoscopes. In some variations, the videoscope and the videoscope interfaces comprise wireless transceivers. In such variations the camera cord 118 (the portion external of the tracheostomy tube 100) and the connector receptacle can be omitted.

The videoscope interfaces may also include configuration connectors (as part of the cable socket) to output image sensor configuration parameters, such as image inversion, clock, shutter speed etc., and to receive configuration information. An I²C protocol may be used to read and/or control the image sensor over data wires extending between the image sensor and the image processor. The data wires do not transmit images, the images (image signals) are transmitted over image wires. If the image sensor has four connectors, for four wires, one of the wires is a data wire, another is an image signal wire, and the remaining two are ground and power wires. The camera cord 118 may include pressure sensor wires to convey the cuff pressure.

As used herein, the term "controller" means a device or devices capable of processing instructions. A controller typically converts coded instructions into timing and control signals that direct the operation of the other components of the device or system, such as memory, arithmetic logic unit, input and output devices, etc. Examples of controllers include complex programmable logic devices (CPLD), central processing units (CPU), graphic processing units (GPU), field programmable gate arrays (FPGAs), a master control unit (MCU) etc. A controller may be a single integrated circuit part or may comprise more than one integrated circuit part. For example, a controller may comprise a combination of a CPU and an FPGA, or a combination of a CPU, a GPU, and an FPGA. The FPGA and GPU may perform graphics processing functions while the MCU performs, as is known, timing and control functions. If the controller comprises more than one integrated circuit part, the integrated circuit parts are linked in a supervised or a distributed manner. For example, a primary integrated circuit part can instruct other integrated circuit parts to execute tasks programmed for the other integrated circuit parts. Alternatively, the other integrated circuit parts may execute their functions independently.

The controller 510 may comprise a CPU 512, an MCU 513, an FPGA 514, and a GPU 516. The CPU 512 performs functions typically performed by CPUs. In the present embodiment a GPU 516 is desired due to the processing requirements of the trained model, including the image processing and the time available to perform the image processing. FPGAs process data very fast compared to other non-volatile memory/instruction combinations and are re-programmable. Therefore, FPGAs facilitate presentation of the live view of the images captured by the endoscope in real-time with minimal latency so that the physician observing the live view can take immediate actions even in emergency situations. The FPGA may process the raw image data generated by the videoscope by performing known optimization functions such as white balance, denoising and the like. The FPGA is optionally provided because it is capable of rapid power-up (i.e. short boot-up time) and thus is useful in emergency situations. As technology evolves, the functionality of the FPGA 514 may be performed without the FPGA 514. The video processor 50 is therefore not limited to the precise packaged integrated circuits described with reference to FIG. 3 but can be constructed to take advantage of design and cost targets and future video processing technologies. For example, faster/more costly memory may be used to increase graphics processing speed. Graphics processing may be provided in the FPGA or a processor that incorporates graphics processing logic, such as a GPU. The image processing circuit 500 comprises processing instructions 515 which may be embedded in the FPGA 514, in the memory 520, or in other memory, such as device embedded memory.

A user interface 518 may be provided in the image processing circuit 500. The user interface 518 may comprise a wireless interface operable to receive user inputs via a mouse, keyboard, or other physical user input devices. Example wireless interfaces include Bluetooth and Zigbee controllers. The user interface 518 may comprise a USB port to receive a USB connector of a wired user input device or a USB wireless interface operable to communicate wirelessly with the mouse, keyboard, and/or other physical user input devices including outputs from the touch display 56. Thus, the video processor 50 provides flexibility in receiving user inputs via various user input devices as is known in the art.

The processing instructions 515 embedded in the memory 520 may comprise a graphical user interface (GUI) logic 522, monitoring logic 524, and processing instructions to perform other image processing steps described below. The memory 520 may comprise multiple interconnected circuits, including a memory circuit embedded in the controller 510, a memory integrated circuit connected to the controller 510, a hard-drive connected to the controller 510, and any other devices operable to store data and communicate with the controller 510. Memory includes volatile and non-volatile memory.

The term "logic" as used herein includes software and/or firmware executing on one or more programmable processing devices, application-specific integrated circuits, field-programmable gate arrays, digital signal processors, hardwired logic, or combinations thereof. Therefore, in accordance with the embodiments, various logic may be implemented in any appropriate fashion and would remain in accordance with the embodiments herein disclosed. Logic may comprise processing instructions embedded in non-transitory machine-readable media (e.g. memory).

The GUI logic 522 comprises processing instructions to generate a GUI 523 presented with or by the video processor 50. The GUI can be responsive to user inputs received via the touch screen or other user inputs. The controller 50 receives video (image data), potentially pre-processed by the FPGA, and outputs video signals incorporating the GUI and image data via an output port 532 of the video output board 530, optionally a High-Definition Multimedia Interface (HDMI) port. The GUI 523 may comprise a record button that can be toggled to record a clip of the live video. The GUI 523 may comprise additional buttons to provide additional functionality. Thus, the controller 510 causes presentation of the live view of the images with the GUI 123.

The monitoring logic 524 is configured to receive pressure data from the pressure sensor, optionally via the wires of the camera cord 118, and to cause the controller 510, which processes the monitoring logic 524 instructions, to output a warning or alarm signal if the pressure falls outside a predetermined cuff pressure range. The range has a lower limit and an upper limit. The upper limit may be predetermined to prevent tissue damage and may be configured to provide a comfortable pressure that does not unduly bother the patient, even over extended use lasting multiple days. The lower limit may be predetermined empirically as the lowest pressure that maintains the tracheostomy tube 100 in its selected position. Thus, the range is configured to maintain the tracheostomy tube 100 in its selected position over extended use while mitigating the risk that it will come lose and move, even when the user is ambulatory.

In one embodiment, a pressure monitoring method comprises: periodically or continuously receiving a pressure signal from the pressure sensor, comparing the pressure signal to the pressure range, and outputting a warning or alarm signal if the pressure signal is indicative of a cuff pressure being outside the pressure range.

The pressure monitoring method may comprise outputting a warning or alarm signal if the pressure signal is lost or the tracheostomy tube 100 is disconnected.

A monitoring system 600 may comprise a monitor 602 including a monitoring logic 604 provided to perform the pressure monitoring method. The pressure monitoring method may also be performed by the image processing circuit 500. The monitoring circuit 604 may be similar but smaller than the image processing circuit 500. As such, the monitoring circuit 604 may be devoid of a display and be configured to be carried by the user, for example attached to the strap or the mounting plate or carried on a belt. The components of the monitoring circuit are selected to minimize weight, size, and power usage. To achieve such resource reductions the controller 510 is configured to monitor pressure signals but not to process images. The image processing circuit 500 may be used to visualize placement of the tracheostomy tube 100, and thereafter the camera cord 118 may be connected to the monitoring circuit 604 to enable the user to mobilize.

The user interface 518 may be provided in the monitoring circuit 500 with a wireless interface operable to receive user inputs and output alarm or warning signals. Example wireless interfaces include Bluetooth and Zigbee controllers, preferably a low-power Bluetooth controller. The user interface 518 may comprise a USB port to recharge batteries.

The monitoring circuit 604 may be devoid of power-hungry image processors, display interfaces, GUI logic, and multiple videoscope interfaces 504. Instead, the monitoring circuit 604 comprises the user interface 518, an efficient controller 510, a battery (rechargeable or not), and memory. The alarm or warning signals 618 may be bluetooth signals output triggered by the monitoring logic 524 and transmitted by the user interface 518 to a paired user device 620, such as a mobile phone of the user. Thus, the monitoring logic 524 may comprise known pairing logic to pair the monitoring circuit 604 with the user's mobile phone or other user device. A monitoring mobile application, or app, in the user's mobile phone may be configured to periodically receive monitoring signals from the monitoring circuit 600, and upon the signals indicating that the cuff pressure is outside the range, sound an alarm, flash a notification and/or transmit a warning or alarm to monitoring system such as a physician's network. Additionally, the monitoring mobile application may transmit a warning or alarm if the paired monitoring circuit 604 fails to communicate in a predetermined manner, which could be indicative of a drained battery or disconnection from the tracheostomy tube 100. The processing instructions 515 embedded in the memory 520 may comprise the monitoring logic 524.

The monitoring circuit 604 may comprise a battery 606 and an alarm device 608, such as a buzzer or vibrator or light configured to illuminate in different colors (green/yellow/red) or flash to indicate an alarm.

## Claims

1. A tracheostomy tube (100) comprising:
a cannula (102) including a proximal end (108), a distal end (110), a wall (102e) extending from the proximal end to the distal end and forming a ventilation lumen (116), the wall (102e) including an outer surface (102a) and an inner surface (102b), the cannula (102) further including a curved section (112), a distal anterior side (102c), a distal posterior side (102d), and an open camera cord channel (144c) extending from the proximal end of the cannula toward the distal end;
a mounting plate (104) positioned at the proximal end of the cannula (102);
a cuff (106) positioned distally of the curved section (112) and extending over a portion of the open camera cord channel (144c);
a cuff inflation lumen (142b) extending from the proximal end to the distal end and configured to inflate the cuff;
a camera assembly (120') positioned distally of the cuff;
a front wall (130b) comprising an opaque portion (132a) and a transparent portion (132b), the transparent portion (132b) positioned distally and in front of the camera assembly;
a fluid lumen (142c) extending from the proximal end to the distal end and configured to supply a fluid to clean the transparent portion of the front wall; and
a camera cord (118) electrically connected to the camera assembly (120'), the camera cord including a camera cord proximal portion (118a) positioned in the open camera cord channel (144c), and a camera cord distal portion (118c) positioned distally of the cuff (106).

2. The tracheostomy tube (100) of claim 1, the camera assembly (120') further comprising a camera housing (130), the camera housing (130) comprising the front wall (130b), and the cannula comprising a camera housing cavity (121, 121c), wherein the camera housing (130) is positioned in the camera cavity (121) and the camera assembly (120') is positioned in the camera housing (130).

3. A tracheostomy tube (100) according to claim 2, wherein the camera housing (130) comprises a discorectangular shape.

4. A tracheostomy tube (100) according to claim 1, the tracheostomy tube (100) further comprising a tip part (140), the tip part (140) comprising a camera cavity (140c), a ventilation lumen (116') extending from the ventilation lumen (116) of the cannula, and the front wall (130b), wherein the camera assembly (120') is positioned in the camera cavity (140c) of the tip part (140).

5. A tracheostomy tube (100) according to any of the preceding claims, wherein the camera cord proximal portion (118a) is radially offset from the camera cord distal portion (118c) by between 30 and 120 degrees.

6. A tracheostomy tube (100) according to any of the preceding claims, wherein the camera cord comprises a relief portion (118d).

7. A tracheostomy tube (100) according to any of the preceding claims, the tracheostomy tube (100) further comprising a nozzle (134) configured to direct the fluid toward the transparent portion (132b) of the front wall (130b).

8. A tracheostomy tube (100) according to any of the preceding claims, further comprising a proximal tip received in a cavity of the wall (102e), the proximal tip being configured to radially align the camera assembly with the cannula.

9. A tracheostomy tube (100) according to any of the preceding claims, the tracheostomy tube (100) further comprising a cuff pressure sensor (150) positioned between the cannula (102) and the cuff (106).

10. A visualization system (10) including the tracheostomy tube (100) according to any of claims 1-7 and a video processor (50), the video processor (50) configured to be communicatively connected with the tracheostomy tube (100) and present images received from the tracheostomy tube (100) on a display (56).

11. The visualization system (10) of claim 10, wherein the tracheostomy tube (100) further comprises a cuff pressure sensor (150) positioned between the cannula (102) and the cuff (106), and wherein the video processor (50) is configured to output an alarm or warning signal when a cuff pressure sensed by the cuff pressure sensor (150) is below a low pressure limit.

12. The visualization system (10) of claim 10, wherein the tracheostomy tube (100) further comprises a cuff pressure sensor (150) positioned between the cannula (102) and the cuff (106), and wherein the video processor (50) is configured to output an alarm or warning signal when a cuff pressure sensed by the cuff pressure sensor (150) is outside a predetermined range.

13. A monitoring system (600) including the tracheostomy tube (100) according to any of claims 1-9 and a monitor (602), wherein the tracheostomy tube (100) further comprises a cuff pressure sensor (150) positioned between the cannula (102) and the cuff (106), wherein the monitor (602) is sized and configured to be carried by a wearer of the tracheostomy tube (100), and wherein the monitor (602) comprises monitoring logic (604) configured to perform a pressure monitoring method comprising:
periodically or continuously receiving a pressure signal from the pressure sensor;
comparing the pressure signal to the pressure range, and
outputting a warning or alarm signal if the pressure signal is indicative of a cuff pressure being outside the pressure range.

14. The monitoring system (600) of claim 13, wherein the warning or alarm signal comprises a Bluetooth signal transmitted to a paired mobile device.
